# EUROPEAN PATENT APPLICATION

(11) **EP 1 964 551 A1**
(43) Date of publication of application: **03.09.2008**
(21) Application number: 08101851.7
(22) Date of filing: 21.02.2008
(51) Int. Cl.: A61K 31/122, A61K 31/525, A61K 36/16, A61P 25/06

(54) **Compositions for the prevention and treatment of primary headache and migraine**

(30) Priority: 01.03.2007 IT MI20070408
(71) Applicant: Pharmaval S.r.L., 36050 Bolzano Vicentino (VI) (IT)
(72) Inventor: D'Andrea, Giovanni, 36050 Bolzano Vicentino (VI) (IT); Rachela, Enrico, 36050 Bolzano Vicentino (VI) (IT)
(74) Representative: Minoja, Fabrizio

(57) **Abstract**

This invention relates to compositions for the prevention and treatment of primary headache and migraine containing pentacyclic diterpenes obtained from Ginkgo biloba, coenzyme Q10 and Vitamin B2.

## Description

This invention relates to compositions for the prevention and treatment of primary headache and migraine containing pentacyclic diterpenes obtained from Ginkgo biloba leaves, coenzyme Q10 and vitamin B2.

### Introduction

Headaches are a very common disorder: in Italy alone, 26 million people, mainly women, apparently suffer from headaches. Headaches are consequently considered a socially significant disorder, which involves a loss of some 200 million hours' work a year.

The term "headache" includes a number of very different disorders, which have been generically classified as primary and secondary headaches on the basis of the quality, intensity and cyclical nature of the pain and the type of onset.

Headache, defined as pain located above a line running from the eye sockets to the base of the nose (the orbitomeatal line), is merely a symptom, which can have multiple causes.

Primary headache is an actual disorder, due to causes which are not always immediately identifiable, but may include some hormonal or environmental trigger factors. Migraines, tension headaches and cluster headaches are the three main forms of primary headache.

Migraine, perhaps the most common form of primary headache, affects around 12% of the world population. Two types of migraine can be distinguished: without aura (85% of cases) and with aura (10-15% of cases).

The tension headache is a form of primary headache characterised by the presence of pain (which feels like a "tight band" or "vice" round the head) of mild to moderate intensity, usually bilateral, which is not exacerbated by physical effort, and is typically not associated with any particular accompanying symptoms. On clinical examination, pain can be evaluated when pressure is exerted on the head muscles. Tension headaches are divided into three types, according to frequency: infrequent, frequent and chronic. The chronic form is a highly disabling headache, and often resistant to treatment.

Finally, cluster headaches are a form of primary headache characterised by attacks of very strong unilateral pain, mainly located at the eye and temple. The attacks can be short-lived, and occur one or more times a day, especially at night. The pain is accompanied by signs and symptoms of activation of the local autonomous nervous system, namely reddening and watering of the eyes, blocked nose or rhinorrhoea, swollen eyelids, ptosis and reduction in pupil diameter, sweating of the face and forehead, in various combinations and on the same side as the pain, together with a feeling of restlessness and/or agitation. There are two clinical variations: an episodic and a chronic form. The episodic form (80-90% of cases) is characterised by periods of activity (clusters) during which the patient has attacks of pain nearly every day, which often occur at specific times (from 1 to 8 times a day), alternating with periods of remission. In the chronic forms, the periods of remission do not last more than a month.

Cluster headache is a rare disorder which affects roughly 1 person in 1000. The prevalence is 3.5 times greater in men, although the number of women affected is constantly increasing. The term "cluster" relates to the characteristic timing of the disorder: the attacks are concentrated in a given period, often with a seasonal recurrence.

The symptomatic treatment of primary headaches must stop the attack in the shortest possible time once it has begun. It must also reduce the associated symptoms, such as nausea and vomiting.

Symptomatic treatment is based on the following categories of drugs:
1) Triptans (Sumatriptan in different formulations: 6 mg subcutaneous, 50 and 100 mg oral, 25 mg rectal and 20 mg nasal spray formulation; Zolmitriptan 2.5 mg per os and lyophilisate; Rizatriptan 10 mg in oral formulation and lyophilisate; Almotriptan 12.5 mg per os; Eletriptan 40 mg per os.
2) NSAIDs and analgesics (ASA, indomethacin, ibuprofen, naproxen, diclofenac, ketoprofen, paracetamol and nimesulide).
3) Ergot derivatives.
   Represented by ergotamine (1-2 mg per os; maximum dose/24 hours: 4 mg; 0.5-2 mg rectally) and dihydroergotamine (1-2 mg spray; maximum dose: 4 mg/24 hours or 12 mg/week; 3-5 mg per os).
4) beta-blockers and calcium antagonists (in this case, their efficacy appears after a few weeks' use).
5) Tricyclic antidepressants and SSRIs.
6) Antiepileptic drugs.

Despite the huge efforts made by the pharmacological industry to treat the various forms of primary headache, the problem is far from solved.

Primary headache is characterised by painful attacks of varying intensity and frequency located on one side or the whole of the head. They are due to sensitisation of the first neurone of the trigeminal vascular system (pulsating oculotemporal pain) followed by sensitisation of the second neurone located in the descending trigeminal nucleus (allodynia and intensification and spread of the pain beyond the trigeminal area). The sensitisation is the result of release of calcitonin gene related peptide (CGRP) by the endings of the first branch of the trigeminal nerve. CGRP causes vasodilatation of the arteries and the release of platelet activating factor (PAF) into the trigeminal vascular system. PAF is a phospholipid mediator which performs numerous biological actions; it stimulates the endothelium to produce nitric oxide, activates the platelets, induces prostaglandin synthesis, and consequently makes a crucial contribution to trigeminal sensitisation and triggers the migraine attack. A pharmacological action that blocks the action of PAF at the relevant sites would be a useful means of prevention and treatment of migraine attacks.

### Description of the invention

It has now been discovered that compositions containing pentacyclic diterpenes extracted from G. biloba leaves, coenzyme Q10 and vitamin B2 are highly effective in the prevention and treatment of primary headache and migraine.

The invention consequently relates to compositions containing the following active constituents:
a) pentacyclic diterpenes obtained from Ginkgo biloba,
b) coenzyme Q10, and
c) vitamin B2
for the prevention and treatment of primary headache and migraine.

It has been found that the compositions according to the invention are highly effective in the prevention and treatment of primary headaches and migraine due to a synergic action which tackles the origin of the primary headache (in all its possible forms) and provides an invaluable method for treating the resulting symptoms.

The combination of the three active ingredients of the compositions according to the invention has an unexpected therapeutic effect due to the synergic action of the three constituents, which counteract the action of the lipid mediator PAF to an unexpectedly great extent.

This activity is even more evident when pentacyclic diterpenes obtained from Ginkgo biloba are used in a form complexed with phospholipids, in particular with soya distearoylphosphatidylcholine, which provides improved oral bioavailability.

Pentacyclic diterpenes (ginkgolide A, B, C, J and M plus their bilobalide metabolite) are "cage-like molecules" which possess strong PAF-antagonist properties.

Coenzyme Q10, or ubidecarenone, is a potent antioxidant and anti-radical with endothelium- and tissue-protecting properties.

Vitamin B2 is a coenzyme of enzymes with metabolic properties responsible for the energy and structural availability of numerous cell compounds.

In accordance with a preferred aspect of the invention, pentacyclic diterpenes obtained from Ginkgo biloba will be present in the compositions in a form complexed with phospholipids, in particular with soya distearoylphosphatidylcholine, as marketed under the brand Phytosome®.

In accordance with a preferred aspect of the invention, the ingredients take the form of phytosome, terclatrate or co-macinate with beta-cyclodextrins or various forms of oil and lipids.

The active ingredients according to the invention will be present in the compositions in the following weight ranges:
a) pentacyclic diterpenes obtained from Ginkgo biloba diterpene fraction phytosome: 0.1 and 360 mg,
b) coenzyme Q10: 0.1-500 mg,
c) vitamin B2: 0.1-250 mg.

According to a particularly preferred aspect, the compositions according to the invention will contain the following basic constituents in the weight ranges shown below:
a) pentacyclic diterpenes obtained from Ginkgo biloba phytosome: 10-60 mg,
b) coenzyme Q10: 10-100 mg,
c) vitamin B2: 0.1-2.5 mg.

The compositions according to the invention could be formulated suitably for oral administration, and will be prepared according to conventional methods well known in pharmaceutical technology, such as those described in Remington's Pharmaceutical Handbook, Mack Publishing Co., N.Y., USA, using excipients, diluents, fillers and anti-caking agents acceptable for their final use. In particular, the compositions according to the invention could be formulated as a suspensible or effervescent powder, ordinary, multilayer, differentiated-release or gastroprotected tablets, medicated gum discs, effervescent tablets or melting powder.

A preferred aspect of the invention is constituted by differentiated-release solid oral forms, especially a slow-release layer bonded to a normal-release layer.

Some examples of formulations are set out below.

### Examples of formulations

### 1) TYPE 1 CAPSULES

| | |
|---|---|
| G. biloba terpenes phytosome | 60 mg |
| Coenzyme Q10 | 10 mg |
| Vitamin B2 | 8 mg |
| Microcel | 41 mg |
| Vegetable magnesium stearate | 7 mg |
| Aerosil 10 | 26 mg |

### 2) 750 mg FILM-COATED TABLETS

| | |
|---|---|
| G. biloba terpenes phytosome | 40 mg |
| Coenzyme Q10 | 10 mg |
| Vitamin B2 | 8 mg |
| Microcel | 340 mg |
| Dicaphos | 333 mg |
| Vegetable magnesium stearate | 10 mg |
| Aerosil | 8 mg |
| Explocel | 18 mg |
| Sepifilm | 22.8 mg |
| Shellac | 7 mg |
| Colouring | 0.2 mg |

### 3) 750 mg TWO-LAYER DIFFERENTIATED-RELEASE TABLETS

### Normal-release layer

| | |
|---|---|
| G. biloba terpenes phytosome | 30 mg |
| Coenzyme Q10 | 5 mg |
| Vitamin B2 | 4 mg |
| Dicaphos | 304 mg |
| Explocel | 15 mg |
| Aerosil | 3 mg |
| Vegetable magnesium stearate | 6 mg |
| Colouring | 1 mg |

### Delayed-release layer

| | |
|---|---|
| G. biloba terpenes phytosome | 30 mg |
| Coenzyme Q10 | 5 mg |
| Vitamin B2 | 4 mg |
| Metholose | 80 mg |
| Aerosil | 2 mg |
| Vegetable magnesium stearate | 3 mg |
| Microcel | 80 mg |
| Dicaphos | 164 mg |

### 4) 2500 mg SACHETS

| | |
|---|---|
| G. biloba terpenes phytosome | 120 mg |
| Coenzyme Q10 | 20 mg |
| Vitamin B2 | 5 mg |
| Fructose | 1873 mg |
| Aerosil | 15 mg |
| Orange flavouring | 180 mg |
| Tangerine flavouring | 40 mg |
| Citric acid | 220 mg |
| Acesulfame K | 25 mg |
| E102 | 2 mg |

### 5) 1800 mg MELTING FORMULATION

| | |
|---|---|
| G. biloba terpenes phytosome | 60 mg |
| Coenzyme Q10 | 10 mg |
| Vitamin B2 | 8 mg |
| Sorbitol | 160 mg |
| Orange flavouring | 20 mg |
| Tangerine flavouring | 5 mg |
| Acesulfame K | 2 mg |
| Aerosil | 5 mg |
| Fructose | 1530 mg |

### 6) 1235 mg GUM DISC

| | |
|---|---|
| G. biloba terpenes phytosome | 60 mg |
| Coenzyme Q10 | 10 mg |
| Vitamin B2 | 8 mg |
| Gum base | 800 mg |
| Aspartame | 2 mg |
| Menthol | 2 mg |
| Acesulfame | 1 mg |
| Levilite | 20 mg |
| Talc F.U. | 20 mg |
| Vegetable magnesium stearate | 18 mg |
| Shellac | 12 mg |
| Xylitol | 250 mg |
| Gum arabic | 6 mg |
| Titanium dioxide | 6 mg |
| Carnauba wax | 0.2 mg |

## Claims

1. Compositions containing Ginkgo biloba pentacyclic diterpenes, coenzyme Q10 and vitamin B2: 0.1-25 mg together with conventional excipients and diluents.

2. Compositions as claimed in claim 1, containing pentacyclic diterpenes obtained from Ginkgo biloba in a form complexed with phospholipids.

3. Compositions as claimed in claim 2, containing pentacyclic diterpenes obtained from Ginkgo biloba in a form complexed with soya distearoylphosphatidylcholine.

4. Compositions as claimed in the preceding claims, wherein the active constituents are present in the following weight ranges:
a) pentacyclic diterpenes obtained from Ginkgo biloba: 0.1-360 mg,
b) coenzyme Q10: 0.1-500 mg,
c) vitamin B2: 0.1-250 mg.

5. Compositions as claimed in the preceding claims, wherein the active constituents are present in the following weight ranges:
a) pentacyclic diterpenes obtained from Ginkgo biloba diterpene phytosome fraction: 10-60 mg,
b) coenzyme Q10: 10-100 mg,
c) vitamin B2: 0.1-2.5 mg.

6. Compositions as claimed in the preceding claims, in the form of a suspensible or effervescent powder, ordinary, multilayer, differentiated-release or gastroprotected tablets, medicated gum discs, effervescent tablets or melting powder.

7. Use of a combination of:
a) pentacyclic diterpenes obtained from Ginkgo biloba,
b) coenzyme Q10, and
c) vitamin B2: 0.1-25 mg,
to prepare a composition for the prevention and treatment of vascular dementia.

8. Use as claimed in claim 7, wherein the pentacyclic diterpenes obtained from Ginkgo biloba are in a form complexed with phospholipids.
